# EUROPÄISCHE PATENTSCHRIFT

(11) **EP 2 585 050 B1**
(45) Veröffentlichungstag und Bekanntmachung des Hinweises auf die Patenterteilung: **14.05.2014**
(21) Anmeldenummer: 11727468.8
(22) Anmeldetag: 27.06.2011
(51) Int. Cl.: A61K 9/48, B82Y 5/00, C08B 37/16, A61K 9/16, A61K 9/20, A61K 31/4045

(54) **SILODOSIN-CYCLODEXTRIN EINSCHLUSSVERBINDUNGEN**
SILODOSIN-CYCLODEXTRINE INCLUSION COMPLEX
COMPLEXE D'INCLUSION DE SILODOSINE ET CYCLODEXTRINE

(30) Priorität: 28.06.2010 EP 10167533
(43) Veröffentlichungstag der Anmeldung: 01.05.2013
(73) Patentinhaber: Ratiopharm GmbH, 89079 Ulm (DE)
(72) Erfinder: RIMKUS, Katrin, 58636 Iserlohn (DE); STEFAN, Ralph, 88370 Ebenweiler (DE)
(74) Vertreter: Keller, Günter
(86) Internationale Anmeldenummer: PCT/EP2011/060691
(87) Internationale Veröffentlichungsnummer: WO 2012/000926

(56) Entgegenhaltungen:
- EP-A1- 1 541 554
- EP-A1- 1 574 215
- EP-A1- 1 806 136
- EP-A1- 1 950 227
- WO-A1-2005/053659
- WO-A1-2007/016757
- US-A1- 2007 129 329

## Beschreibung

Die Erfindung betrifft ein Arzneimittel mit einer Einschlussverbindung aus Silodosin bzw. einem pharmazeutisch verträglichen Salz davon und einem Cyclodextrin sowie ein Verfahren zur Herstellung des Arzneimittels.

Silodosin ist der internationale Freiname der Verbindung 1-(3-hydroxypropyl)-5-[(2R)-({2-[2-[2-(2,2,2-trifluorethoxy)phenoxy]ethyl}amino)propyl]indolin-7-carboxamid mit der folgenden chemischen Strukturformel:

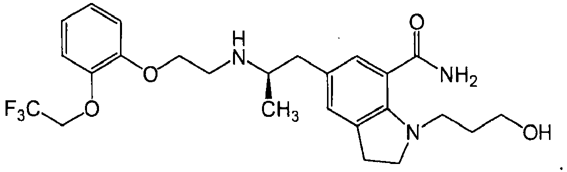

Silodosin ist ein bekannter Arzneistoff, der zur Behandlung der Symptome von benigner Prostatahyperplasie (einer gutartigen Vergrößerung der Prostatadrüse) verwendet wird. Insbesondere ist Silodosin ein α₁-Adrenorezeptorantagonist, der die in der Prostatadrüse lokalisierten Rezeptoren blockiert und zu einer Relaxation der glatten Muskulatur des Blasenhalses, der Harnröhre und der Prostata, verbunden mit einem erleichterten Harnabfluss führt. Silodosin wird daher insbesondere auch zur Behandlung der Dysurie eingesetzt. Weiterhin wurde Silodosin als Empfängnisverhütungsmittel für Männer vorgeschlagen.

Die Herstellung von Silodosin wird beispielsweise in dem Grundpatent EP 0 600 675 offenbart, in dem auch die Verwendung von Silodosin zur Behandlung der Dysurie beschrieben wird. Silodosin-enthaltende Arzneimittel sind derzeit kommerziell erhältlich, unter anderem unter den Marken Urief^{®} und Rapaflo^{®}. Bei den derzeit vertriebenen Arzneimitteln handelt es sich um Hartgelatinekapseln.

Bei der Herstellung von Arzneimitteln mit dem Wirkstoff Silodosin ergeben sich eine Reihe von Schwierigkeiten, da Silodosin als lichtempfindlich gilt und auch keinen hohen Temperaturen ausgesetzt werden soll. Ferner ist Silodosin mit einer Reihe von pharmazeutisch verträglichen Zusatzstoffen, die häufig bei der Herstellung von Arzneimitteln eingesetzt werden, nicht verträglich und bei der Herstellung und Lagerung von Silodosin-haltigen Arzneimittel entstehen daher häufig unerwünschte Abbauprodukte. Entsprechende Probleme werden beispielsweise in der EP-A 1 574 215 beschrieben (in der für Silodosin die ältere Bezeichnung KMD-3213 verwendet wird). Weiterhin treten ausweislich der EP 1 574 215 Probleme bei der Löslichkeit von Tabletten mit dem Wirkstoff Silodosin auf und es ist ausweislich der EP-A 1 574 215 auch schwierig, Silodosin mit einem Schmiermittel zu formulieren. Bei der Herstellung von Kapseln mit dem Wirkstoff Silodosin soll die Mischungszeit des Kapselinhalts einen Einfluss auf das Freisetzungsprofil des Wirkstoffs haben.

Zur Lösung dieser Probleme offenbart die EP-A 1 574 215 einige spezielle Arzneimittel, insbesondere solche, bei denen als Füllmittel D-Mannit und als Schmiermittel Magnesiumstearat vorgesehen ist.

Die JP 2004-175796 offenbart im Mund zerfallende Tabletten und betrifft insbesondere die Geschmacksmaskierung von schlecht schmeckenden Wirkstoffen. Silodosin (bzw. KMD-3213) ist einer von mehreren genannten Wirkstoffen.

Bei der Herstellung von Arzneimitteln mit dem Wirkstoff Silodosin tritt ebenfalls die Schwierigkeit auf, dass Silodosin und insbesondere auch die Salze des Silodosins in amorpher, aber auch in verschiedenen polymorphen kristallinen Formen vorliegen können, wie es beispielsweise der EP 1 541 554 beschrieben ist. In Abhängigkeit von der Form ist Silodosin mehr oder weniger hygroskopisch. Es kann auch nicht ausgeschlossen werden, dass es während der Herstellung und der Lagerung eines Arzneimittels mit dem Wirkstoff Silodosin oder einem pharmazeutisch verträglichen Salz davon zu einer (teilweisen) Umwandlung der polymorphen Formen ineinander oder in die amorphe Form kommt, was Einfluss auf die pharmacokinetischen Eigenschaften des Arzneimittels, insbesondere die Wirkstofffreisetzung und die Bioverfügbarkeit des Wirkstoffs haben kann und auch die Hygroskopizität des Arzneimittels beeinflusst.

Dies erhöht die Probleme, die bei der Formulierung von Arzneimitteln auftreten und macht es schwierig, Arzneimittel mit einer reproduzierbaren und schnellen Wirkstofffreisetzung zur Verfügung zu stellen, die darüber hinaus noch einfach mit einer großen Vielzahl von Hilfsstoffen und kostengünstig erhalten werden können.

Ferner soll das Silodosin in dem Arzneimittel auch eine sehr hohe chemische Stabilität aufweisen und es sollen sich bei der Herstellung und Lagerung des Arzneimittels möglichst wenig Abbauprodukte des Silodosins bilden.

Aufgabe der vorliegenden Erfindung ist es daher, ein Arzneimittel zur Verfügung zu stellen, das die Probleme des Standes der Technik nicht zeigt, das insbesondere, unabhängig von der polymorphen Form des zu seiner Herstellung eingesetzten Silodosins bzw. eines pharmazeutisch verträglichen Salzes davon, reproduzierbar eine sehr schnelle Auflösung und Wirkstofffreisetzung und gute Bioverfügbarkeit sowie eine sehr hohe chemische Stabilität des Silodosins bereitstellt. Das Arzneimittel soll einfach und kostengünstig hergestellt werden können und eine ausgezeichnete Lagerbeständigkeit aufweisen.

Diese Aufgabe wird durch den Gegenstand der Patentansprüche gelöst.

Die Lösung der Aufgabe beruht auf dem überraschenden Befund, dass Silodosin bzw. dessen Salze mit Cyclodextrinen Einschlussverbindungen bildet, die direkt zu Arzneimitteln verarbeitet werden können. Die Arzneimittel mit der Silodosin-Cyclodextrin Einschlussverbindung weisen die Probleme des Standes der Technik nicht auf. Insbesondere ist das Silodosin in diesem Arzneimittel chemisch sehr stabil und die Einschlussverbindung ist mit einer sehr großen Anzahl üblicher pharmazeutischer Zusatzstoffe ohne Probleme zu einem Arzneimittel verarbeitbar. Die physikalische Form des Silodosins in dem Arzneimittel ändert sich auch während der Lagerung nicht und Arzneimittel mit der erfindungsgemäßen Einschlussverbindung weisen eine reproduzierbare, sehr schnelle Freisetzung des Wirkstoffes auf, die sich auch während der Lagerung des Arzneimittels nicht signifikant verändert. Zur Herstellung des erfindungsgemäßen Arzneimittels kann Silodosin oder ein Salz davon eingesetzt werden, und die Eigenschaften des erfindungsgemäßen Arzneimittels sind unabhängig davon, ob der Wirkstoff bzw. das Wirkstoffsalz in amorpher Form oder in kristalliner Form oder in einer bestimmten polymorphen Form verwendet wurde. Schließlich ist das Verfahren ausgesprochen ökonomisch und einfach durchzuführen und bietet dem Galeniker eine große Freiheit bei der Wahl der möglichen pharmazeutisch verträglichen Hilfs- und Zusatzstoffen.

Die erfindungsgemäßen Arzneimittel zeigen eine ausgezeichnete Lagerstabilität. Insbesondere liegt die Menge an Abbauprodukten des Silodosins bei einer Lagerung des Arzneimittels über 12 Monate bei 25°C und 60% Luftfeuchte in der Regel unter 2 Gew.-%, bevorzugt unter 1 Gew.-%, bezogen auf die Menge des Silodosins.

Bei dem erfindungsgemäßen Verfahren wird Silodosin oder ein pharmazeutisch verträgliches Salz davon mit einem Cyclodextrin in Gegenwart eines Lösemittels in Kontakt gebracht. Hierdurch entsteht eine Silodosin-Cyclodextrin Einschlussverbindung. Die Silodosin-Cyclodextrin Einschlussverbindung wird dann mit einem oder mehreren pharmazeutisch verträglichen Zusatzstoffen gemischt. Das Verfahren umfasst ebenfalls mindestens einen Trocknungsschritt. Das Gemisch kann als solches eingesetzt werden, bevorzugt wird das Gemisch jedoch gegebenenfalls mit einem oder mehreren weiteren pharmazeutisch verträglichen Zusatzstoffen zu einer Einheitsdosisform, insbesondere einer Tablette oder einer Hartgelatinekapsel weiterverarbeitet.

Bei einem erfindungsgemäß bevorzugten Verfahren wird Silodosin oder ein pharmazeutisch verträgliches Salz davon zusammen mit Cyclodextrin in ein geeignetes Lösemittel eingebracht. Hierbei kann zunächst das Silodosin oder das pharmazeutisch verträgliche Salz davon zu dem Lösemittel gegeben werden und anschließend das Cyclodextrin zugegeben werden oder es kann zunächst das Cyclodextrin in einem Lösemittel vorgelegt werden und anschließend das Silodosin oder das pharmazeutisch verträgliche Salz davon zugegeben werden. Alternativ können auch Cyclodextrin und Silodosin bzw. ein pharmazeutisch verträgliches Salz davon gleichzeitig zu dem Lösemittel zugegeben werden. Das Gemisch wird homogenisiert wobei die Silodosin-Cyclodextrin Einschlussverbindung entsteht. Das Lösemittel liegt hier bevorzugt im Überschuss vor, so dass eine Suspension oder Lösung entsteht. Die Suspension oder Lösung wird dann auf ein Füllmittel gegeben, gegebenenfalls werden weitere pharmazeutisch verträgliche Zusatzstoffe zugegeben und es wird granuliert. Das Granulat wird getrocknet und gegebenenfalls mit weiteren pharmazeutisch verträglichen Zusatzstoffen gemischt. Das so erhaltene Produkt kann als solches verwendet werden, bevorzugt wird es aber zu einer Einheitsdosisform weiterverarbeitet, insbesondere zu einer Tablette oder Hartgelatinekapsel.

Bei einem weiteren erfindungsgemäß bevorzugten Verfahren wird zunächst das Cyclodextrin mit einem Lösemittel zu einer Paste verarbeitet. Das Silodosin oder das pharmazeutisch verträgliche Salz davon wird dann zu der Paste gegeben und mit der Paste homogenisiert. Hierbei bildet sich die Silodosin-Cyclodextrin Einschlussverbindung. Alternativ dazu kann selbstverständlich auch Silodosin und Cyclodextrin zusammen mit einem Lösemittel zu einer Paste verarbeitet werden. Die homogenisierte Paste wird getrocknet und auf eine gewünschte Teilchengröße gesiebt und anschließend mit mindestens einem pharmazeutisch verträglichen Zusatzstoff gemischt. Wiederum kann eine Weiterverarbeitung zu einer Einheitsdosisform erfolgen, z.B. einer Tablette oder Hartgelatinekapsel.

Ein großer Vorteil des erfindungsgemäßen Verfahrens liegt darin, dass es keine Rolle spielt ob das Silodosin in amorpher Form oder in kristalliner (polymorpher) Form verwendet wird. Auch pharmazeutisch verträgliche Salze des Silodosins können in jeder beliebigen Form eingesetzt werden. Unter dem Begriff Silodosin und pharmazeutisch verträgliche Salze davon, wie er im Rahmen dieser Anmeldung verwendet wird, werden selbstverständlich auch alle Hydrate und Solvate der entsprechenden Verbindungen verstanden. Bevorzugte pharmazeutisch verträgliche Salze des Silodosins, die erfindungsgemäß verwendet werden können, sind Säureadditionssalze. Beispiele für geeignete Säureadditionssalze sind Hydrochloride, Maleate, Oxalate, Sulfate, Methansulfonate, Isothionate, Toluolsulfonate, Trifluoracetate, Pyruvate, Sulfamide, Glutamate, Glutarate, Sorbate und/oder Succinate. Bevorzugt wird allerdings Silodosin in Form der freien Base verwendet.

Cyclodextrine sind eine Klasse von Verbindungen, die zu den cyclischen Oligosacchariden gehören. Sie stellen ringförmige Abbauprodukte von Stärke dar. Der Ring aus sechs Glucoseeinheiten wird auch als α-Cyclodextrin bezeichnet, der Ring aus sieben Glucoseeinheiten wird als β-Cyclodextrin bezeichnet und der Ring aus acht Glucoseeinheiten wird als γ-Cyclodextrin bezeichnet. Es ist bekannt, dass Cyclodextrine mit bestimmten anderen Molekülen Komplexe bilden können. Man nennt solche Komplexe häufig Cyclodextrineinschlussverbindungen. Für die Ausbildung eines Komplexes zwischen Cyclodextrin und einer weiteren Verbindung müssen mehrere Bedingungen erfüllt sein. So muss das Molekül, das die Einschlussverbindung mit dem Cyclodextrin bilden soll, mit seiner Größe und seiner Geometrie in den Hohlraum des Cyclodextrins passen und intermolekulare Wechselwirkungen zwischen dem Cyclodextrin und dem eingeschlossenen Molekül müssen möglich sein. Es ist erfindungsgemäß überraschend, dass Silodosin und pharmazeutisch verträgliche Salze davon mit Cyclodextrinen Einschlussverbindungen bilden, die dann direkt zu einem Arzneimittel mit den hervorragenden Eigenschaften verarbeitet werden können, wie es erfindungsgemäß aufgefunden wurde.

Erfindungsgemäß können α-, β- und γ-Cyclodextrin entweder alleine oder im Gemisch miteinander verwendet werden. Erfindungsgemäß bevorzugt handelt es sich bei dem Cyclodextrin um γ-Cyclodextrin.

Das Verhältnis von Cyclodextrin zu Silodosin ist nicht besonders eingeschränkt, allerdings ist der Anteil an Cyclodextrin in der Regel mindestens so hoch wie der Anteil an Silodosin. Bevorzugt liegt das molare Verhältnis von Cyclodextrin zu Silodosin (bzw. dem Salz davon) im Bereich von 1:1 1 bis 10:1, stärker bevorzugt im Bereich von 1:1 1 bis 5:1.

Überraschenderweise erfolgt der Einschluss des Silodosins in das Cyclodextrin in einem ganz erheblichen Ausmaß. So werden erfindungsgemäß bevorzugt mindestens 85% des Silodosins bzw. des Silodosinsalzes in das Cyclodextrin eingebettet, so dass eine Einschlussverbindung vorliegt, bevorzugt 90% des Silodosins bzw. des Salzes davon. Das heißt, dass in dem erfindungsgemäßen Arzneimittel bevorzugt nur 15% oder weniger, stärker bevorzugt nur 10% oder weniger des Silodosins in freier Form, d.h. in ungeordnetem amorphem Zustand oder in kristallinem Zustand vorliegen. Die vorstehenden Zahlen beziehen sich auf Mol-%. Besonders bevorzugt liegt das Silodosin bzw. das Salz davon vollständig als Einschlussverbindung vor.

Die Cyclodextrineinschlussverbindung mit dem Silodosin kann qualitativ und quantitativ über bekannte Methoden zur Bestimmung von Cyclodextrin Einschlussverbindungen, beispielsweise über XRD nachgewiesen werden. Die Einschlussverbindung wird hierbei durch das Auftreten von Halo-Wellen detektiert. Falls die Cyclodextrin Einschlussverbindung zu 100% vorliegt, werden dabei ausschließlich Halo-Wellen detektiert, charakteristische Peaks von kristallinem Silodosin sind nicht nachweisbar. Falls noch Restanteile von kristallinem Silodosin vorhanden sind, sind die charakteristischen Peaks von kristallinem Silodosin entsprechend reduziert.

Bevorzugt wird die Silodosin-Cyclodextrin Einschlussverbindung jedoch über Dynamische Differenzkalorimetrie (DSC, *Differential Scanning Calorimetry*) nachgewiesen. Die Dynamische Differenzkaloriemetrie ist ein Verfahren zur Messung der abgegebenen bzw. aufgenommenen Wärmemenge einer Probe bei isothermer Arbeitsweise.

Die dynamische differenzkalorimetrische Messung kann z.B. mit einem DSC STARe von Mettler Toledo durchgeführt werden. Die Messung erfolgte für den Messbereich zwischen 25-150°C mit einer Meßgeschwindigkeit von 10°C/Min.

Die Komplexbildung wird über einen Zeitraum von mindestens zwei Stunden, bevorzugt mindestens vier Stunden, stärker bevorzugt mindestens sechs Stunden verfolgt. Die Probenentnahme erfolgt üblicherweise in festgelegten Zeiträumen wie alle 15 Minuten, alle 30 Minuten oder alle Stunde. Für die DSC Messung wird eine definierte Menge des Reaktionsansatzes entnommen und zur Trockene eingeengt. Als Referenzprobe dient eine mechanisch vermischte äquimolare Menge der im Reaktionsansatz eingesetzten Substanzen, die jedoch nicht mit einem Lösemittel in Kontakt gebracht wurden. Die Silodosin-Cyclodextrin Einschlussverbindung zeigt gegenüber der Referenzprobe eine Differenz in der aufgenommenen bzw. abgegebenen Wärmemenge; bzw. eine Differenz in der Umwandlungstemperatur.

Erfolgt die Bildung der Silodosin-Cyclodextrin Einschlussverbindung in einer Paste, kann die Bildung der Silodosin-Cyclodextrin Einschlussverbindung auch mittels Viskosimetrie bestimmt werden. Durch die Bildung der Silodosin-Cyclodextrin Einschlussverbindung in der Paste kommt es zu einer Veränderung der Viskosität, deren zeitlicher Verlauf bestimmt werden kann und die ein Maß für die Bildung der Einschlussverbindung darstellt.

Die Viskositätsmessung kann beispielsweise mit dem ViscoTester® 550 mit Platte-Kegel-System der Firma Thermo Haake nach ISO 3219 Norm erfolgen.

Die Wahl des Lösemittels ist nicht besonders eingeschränkt, hierfür bieten sich alle Lösemittel an, die das Silodosin oder das Silodosinsalz und das Cyclodextrin in ausreichendem Maße lösen, so dass sich die Silodosin-Cyclodextrin Einschlussverbindung bilden kann, die bevorzugt ebenfalls in dem Lösemittel löslich ist. Gegebenenfalls kann zur Verbesserung der Löslichkeit der Verbindungen in dem Lösemittel ein Tensid zugesetzt werden. Bevorzugt sind pharmazeutisch verträgliche Lösemittel und besonders bevorzugt sind C₁-C₄-Alkohole, gegebenenfalls im Gemisch mit Wasser und C₁-C₄-Alkohole. Ganz besonders bevorzugt wird als Lösemittel Ethanol, Wasser oder ein Ethanol-Wasser-Gemisch verwendet, wobei in dem Alkohol-Wasser-Gemisch, bevorzugt dem Ethanol-Wasser-Gemisch jeder der Bestandteile im Bereich von 10-90% vorliegen kann, am stärksten bevorzugt im Bereich von 70-30%, am stärksten bevorzugt bei etwa 50%.

In einer ersten bevorzugten Ausführungsform wird zur Bildung der Silodosin-Cyclodextrin Einschlussverbindung so viel Lösemittel eingesetzt, dass sich eine Lösung oder eine Suspension bildet. Bei dieser Ausführungsform wird das Gemisch mit dem Lösemittel, dem Silodosin oder dem Salz davon und dem Cyclodextrin dann homogenisiert, wodurch sich die Silodosin-Cyclodextrin Einschlussverbindung bildet.

In Abhängigkeit der Menge des Silodosins, der Art und Menge des Silodosinsalzes und der Menge des Cyclodextrins sowie des verwendeten Lösemittels, der Temperatur und des pH-Wertes kann es vorteilhaft sein, dem Gemisch aus Cyclodextrin und Wirkstoff noch ein Tensid zuzugeben. Das Tensid ist nicht besonders eingeschränkt. Als Beispiel können Polysorbat 80, Natriumlaurylsulfat, Polysorbat 20, Polyalkylenglycolether etc. genannt werden. Besonders bevorzugt ist Natriumlaurylsulfat.

Erfindungsgemäß bevorzugt erfolgt bei dieser ersten bevorzugten Ausführungsform das Homogenisieren durch intensives Rühren des Gemisches, allerdings kann auch ein Ultraturrax für die Homogenisierung verwendet werden. Bevorzugt erfolgt also die Bildung der Silodosin-Cyclodextrin Einschlussverbindung dadurch, dass Silodosin oder ein pharmazeutisch verträgliches Salz davon, das Cyclodextrin und gegebenenfalls ein Tensid in ein geeignetes Lösemittel wie vorstehend erläutert eingebracht und homogenisiert wird.

Bei dieser bevorzugten Ausführungsform des erfindungsgemäßen Verfahrens wird die Lösung oder Suspension mit der Silodosin-Cyclodextrin Einschlussverbindung bevorzugt direkt auf ein geeignetes pharmazeutisch verträgliches Füllmittel aufgebracht. Es ist im Prinzip auch möglich, zunächst das Lösemittel zu entfernen und damit die Silodosin-Cyclodextrin Einschlussverbindung zu isolieren und dann die isolierte Silodosin-Cyclodextrin Einschlussverbindung mit dem Füllmittel zu granulieren, dies ist aber erfindungsgemäß nicht bevorzugt. Die erfindungsgemäßen Vorteile werden am Besten dadurch erreicht, dass die in dem Lösemittel homogenisierte Silodosin-Cyclodextrin Einschlussverbindung direkt mit dem Lösemittel auf das Füllmittel aufgebracht wird.

Das Lösungsmittel kann beispielsweise durch Lyophilisierung, Sprühtrocknung oder Vakuumtrocknung entfernt werden.

Nach dem Auftragen der Lösung oder Suspension mit der Silodosin-Cyclodextrin Einschlussverbindung auf das Füllmittel wird das Gemisch erfindungsgemäß bevorzugt granuliert. Gegebenenfalls kann dem Füllmittel mit der Silodosin-Cyclodextrin Einschlussverbindung vor dem Granulieren noch ein oder mehrere weitere übliche pharmazeutische Hilfs- und Zusatzstoffe beigegeben werden. Diese Hilfs- und Zusatzstoffe werden nachstehend noch näher erläutert. Erfindungsgemäß bevorzugt wird daher das Füllmittel mit der Lösung der Silodosin-Cyclodextrin Einschlussverbindung und gegebenenfalls dem Tensid auf bekannte Art und Weise granuliert.

Das Granulieren erfolgt in üblichen Vorrichtungen zum Granulieren, z.B. Diosna P1/6, DIOSNA Dierks & Söhne GmbH. Bevorzugt erfolgt die Granulierung derart, dass das Granulat eine gewichtsgemittelte Teilchengröße (D50-Wert) im Bereich von 75-800 µm aufweist, stärker bevorzugt im Bereich von 100-500 µm. Die gewichtsgemittelte Teilchengröße kann beispielsweise mittels Siebanalyse zum Beispiel unter Verwendung einer Retsch^{®} AS 2000 Vorrichtung bestimmt werden, wobei eine Amplitude von 1,5 Sekunden, ein Intervall von 10 Minuten und eine Probeneinwaage von 15,8 g als beispielhafte Messbedingung angegeben werden können.

Das erhaltene Granulat kann dann auf übliche Art und Weise getrocknet werden, wobei die Trocknung bevorzugt so erfolgt, dass der Lösemittelgehalt im Granulat maximal 0,5%, stärker bevorzugt maximal 0,3% beträgt. Dies ist auch der bevorzugte Gehalt an Restlösemittel in dem erfindungsgemäßen Arzneimittel. Die Trocknung kann in einem üblichen Trockenofen, bevorzugt unter vermindertem Druck bei einer Temperatur im Bereich von 20-80°C, bevorzugt im Bereich von 20-50°C erfolgen. Da das Silodosin in den erfindungsgemäßen Cyclodextrin-Silodosin Einschlussverbindungen eine ausgesprochen hohe Stabilität aufweist, ist die Wahl der Trocknungsbedingungen nicht besonders eingeschränkt. Beispielsweise ist eine Änderung der polymorphen Form gar nicht und eine Zersetzung des Wirkstoffs nur bei Trocknung für längere Zeit unter sehr hohen Temperaturen zu befürchten.

Nach dem Trocknen erhält man das erfindungsgemäße Arzneimittel.

Bei einer zweiten bevorzugten Ausführungsform erfolgt die Homogenisierung und die Bildung der Silodosin-Cyclodextrin Einschlussverbindung in einer Paste wie vorstehend bereits beschrieben. Die Paste wird bevorzugt getrocknet, gesiebt und anschließend bevorzugt auf ein geeignetes pharmazeutisch verträgliches Füllmittel aufgebracht und granuliert. Es ist auch möglich, die Paste direkt auf ein geeignetes pharmazeutisch verträgliches Füllmittel aufzubringen und das Lösemittel anschließend zu entfernen, dies ist aber erfindungsgemäß nicht bevorzugt.

Als Füllmittel kann im Prinzip jedes bekannte pharmazeutisch verträgliche Füllmittel verwendet werden. Beispiele für erfindungsgemäß bevorzugte Füllmittel sind Calciumcarbonat. Magnesiumcarbonat, Magnesiumoxid, Calciumsulfat, Stärke, Stärkederivate, modifizierte Stärke, Polysaccharide, Chitin, Cellulose, Cellulosederivate, Calciumphosphat, Calciumhydrogenphosphat, Saccharose, Maltodextrin, Dextrat, Dextrin, Dextrose, Laktose, Laktosederivate und hydrogeniertes Pflanzenöl. Erfindungsgemäß bevorzugt sind Calciumphosphat, Calciumhydrogenphosphat und Polysaccharide, insbesondere Sojapolysaccharid, das beispielsweise unter der Marke Emcosoy^{®} vertrieben wird.

Die Menge an Füllmittel ist üblicherweise deutlich höher als die Menge an Cyclodextrin und Silodosin bzw. Silodosinsalz. In der Regel enthält das erfindungsgemäße Arzneimittel (nach dem Entfernen des Lösemittels) 40-96 Gew.-% Füllstoff, 1-10 Gew.-% Silodosin bzw, ein pharmazeutisch verträgliches Salz des Silodosins, 3-50 Gew.-% Cyclodextrin und 0-1 Gew.-% Tensid. In der folgenden Tabelle sind bevorzugte Gehalte an Silodosin, Cyclodextrin, Füllmittel und gegebenenfalls Tensid aufgeführt.

| | | | | |
|---|---|---|---|---|
| Silodosin oder pharmazeutisch verträgliches Salz davon | 1-8 | 1-7 | 1-5 | 2-5 |
| Cyclodextrin | 4-45 | 4-40 | 4-35 | 4-35 |
| Füllmittel | 46-95 | 52-95 | 60-95 | 60-94 |
| Tensid | 0-1 | 0-1 | 0-0,5 | 0-0,5 |

Die vorstehenden Angaben sind in Gew.-%, bezogen auf das erfindungsgemäße Arzneimittel ohne möglichen Restlösemittelgehalt.

Das erfindungsgemäße Arzneimittel kann als solches verwendet werden, das heißt direkt an einen Patienten verabreicht werden, bevorzugt wird es aber zunächst in eine Einheitsdosisform überführt, die zur oralen Verabreichung geeignet und üblich ist. Geeignete Einheitsdosisformen sind beispielsweise Sachets, Kapseln, Tabletten oder Suppositorien. Erfindungsgemäß sind Einheitsdosisformen zur oralen Verabreichung bevorzugt. Besonders bevorzugt sind erfindungsgemäß Tabletten und Kapseln, wobei unter Kapseln insbesondere Hartgelatinekapseln verstanden werden. Erfindungsgemäß bevorzugt handelt es sich bei dem Arzneimittel um eine Kapsel oder eine Tablette, am stärksten bevorzugt um eine Tablette oder eine Hartgelatinekapsel.

Hierzu kann das Granulat bzw. das Gemisch, das die Silodosin-Cyclodextrin Einschlussverbindung enthält beispielsweise direkt und ohne weitere Hilfs- und Zusatzstoffe zum Beispiel in ein Sachet oder eine Kapsel überführt oder zu einer Tablette verpresst werden oder es kann zunächst mit geeigneten weiteren Hilfs- und Zusatzstoffen gemischt und anschließend beispielsweise in ein Sachet oder eine Kapsel abgefüllt oder zu einer Tablette verpresst werden. Geeignete Hilfs- und Zusatzstoffe sind dem Fachmann bekannt und beispielsweise im Europäischen Arzneibuch beschrieben. Weitere mögliche Hilfsstoffe sind beispielsweise Sprengmittel, Trennmittel, weitere Füllstoffe, Bindemittel, Zusätze zur Verbesserung der Pulverfließfähigkeit, Schmiermittel, Fließregulierungsmittel und/oder Trennmittel.

Besonders bevorzugt enthält das erfindungsgemäße Arzneimittel mindestens ein Sprengmittel, bevorzugt in einem Gehalt im Bereich von 1-10, bevorzugt 3-6 Gew.-%. Ein Sprengmittel beschleunigt den Zerfall einer Darreichungsform, insbesondere einer Tablette, nach dem Einbringen in Wasser oder in Kontakt mit den Verdauungssäften.

Erfindungsgemäß bevorzugte Sprengmittel sind organische Sprengmittel, wie Carageenan, Croscarmelose, Natriumcarboxymethylstärke, Soja, Polysaccharide und Crospovidon. Erfindungsgemäß bevorzugt enthält das erfindungsgemäße Arzneimittel als Sprengmittel ein sogenanntes "Supersprengmittel", zu denen insbesondere quervernetztes PVP und quervernetztes Natriumstärkeglycolat zählen. Besonders bevorzugt wird erfindungsgemäß quervernetzte PVP als Sprengmittel verwendet.

Schmiermittel dienen im Allgemeinen zur Verringerung der Gleitreibung, was insbesondere bei der Tablettierung vorteilhaft sein kann. Erfindungsgemäß bevorzugte Schmiermittel sind Stearinsäure, Adipinsäure, Natriumstearylfumarat (beispielsweise unter der Marke Pruv^{®} vertrieben) und/oder Magnesiumstearat. Der Gehalt von Schmiermittel beträgt bevorzugt 0-5, stärker bevorzugt 0,1-5, insbesondere 0,5-3 Gew.-%.

Weiterhin können die erfindungsgemäßen Arzneimittel auch noch beispielsweise Bindemittel enthalten. Beispiele für Bindemittel sind Copovidon, Gelatine und Hydroxypropylcellulose. Andere Bindemittel sind dem Fachmann bekannt. Der Gehalt an Bindemittel ist bevorzugt 0-10, insbesondere 0,5-10 Gew.-%.

Viele Verbindungen zählen sowohl zu der Gruppe der Sprengmittel als auch zur Gruppe der Bindemittel, beispielsweise mikrokristalline Cellulose. Eine solche Verbindung kann selbstverständlich ebenfalls in den erfindungsgemäßen Arzneimitteln vorhanden sein und dort je nach Konzentration und übrigen Bestandteilen die Funktion eines Bindemittels oder eines Sprengmittels aufweisen.

Ein erfindungsgemäßes Arzneimittel kann ferner noch Zusätze zur Verbesserung der Fließfähigkeit enthalten, bevorzugt in einer Menge von 0-5, insbesondere von 1,5-4 Gew.-%. Ein Beispiel für einen derartigen Hilfsstoff ist disperses Siliziumdioxid, das beispielsweise unter dem Handelsnamen Aerosil^{®} vertrieben wird. Bevorzugt wird Siliziumdioxid mit einer spezifischen Oberfläche von 50-400 m²/g, bestimmt nach Gasabsorption gemäß PH Eur., 6. Auflage, 2.9.26.

Als geeignete Tenside können die gleichen Verbindungen angegeben werden, die bereits vorstehend genannt wurden.

Die erfindungsgemäßen Arzneimittel können weiterhin beispielsweise auch noch Trennmittel enthalten. Unter Trennmitteln werden erfindungsgemäß im Einklang mit dem allgemeinen Fachwissen Stoffe verstanden, welche die Agglomeration im Kernbett vermindern. Beispiele für geeignete Trennmittel sind Talk, Silicagel, Polyethylenglykol (bevorzugt mit 2000-10000 g/mol gewichtsmittlerem Molekulargewicht) und/oder Glycerolmonostearat.

In der folgenden Tabelle werden bevorzugte Zusammensetzungen und Konzentrationen von erfindungsgemäßen Arzneimitteln offenbart. Alle Angaben der Tabelle sind in Gewichtsprozent, bezogen auf das fertige Arzneimittel angegeben (ohne Berücksichtigung von gegebenenfalls vorhandenem Restlösemittel).

| | | | | | |
|---|---|---|---|---|---|
| Silodosin oder pharmazeutisch verträgliches Salz davon | 1-8 | 1-5 | 1-5 | 1-5 | 2-5 |
| Cyclodextrin | 1-50 | 1-50 | 5-50 | 5-50 | 5-50 |
| Füllmittel | 40-95 | 40-94 | 40-90 | 40-90 | 40-90 |
| Tensid | 0-0,5 | 0-0,5 | 0-0,5 | 0-0,5 | 0-0,5 |
| Sprengmittel | 1-10 | 3-6 | 1-10 | 3-6 | 3-6 |
| Schmiermittel | 0-5 | - | 0-5 | 0,5-4 | 0,5-4 |
| Bindemittel | 0-10 | - | 0-10 | 0-10 | 0-10 |
| Mittel zur Verbesserung der Fließfähigkeit | 0-5 | - | 0-5 | 0,5-3 | 0,5-3 |
| Trennmittel | 0-5 | - | 0-5 | 0-3 | 0-3 |

Das Vermischen der üblichen pharmazeutisch verträglichen Hilfsstoffe mit dem Granulat bzw. dem Gemisch, das die Silodosin-Cyclodextrin Einschlussverbindung enthält, zur Herstellung einer Einheitsdosisform kann in üblicher Art und Weise in handelsüblichen Mischern erfolgen, beispielsweise kann ein Turbula^{®} T10B (Bachhofen AG, Schweiz) genannt werden. Die Mischzeit in handelsüblichen Mischern beträgt üblicherweise 1 Minute bis 1 Stunde, bevorzugt 5 Minuten bis 20 Minuten.

In einer erfindungsgemäß bevorzugten Ausführungsform werden die erfindungsgemäßen Granulate bzw. Gemische gegebenenfalls nach Zusatz von weiteren pharmazeutisch verträglichen Hilfs- und Zusatzstoffen zu Tabletten verpresst. Die Kompression kann mit im Stand der Technik bekannten Tablettiermaschinen wie Exzenterpressen oder Rundlaufpressen erfolgen. Im Fall von Rundlaufpressen wird üblicherweise eine Presskraft von 2-40 kN, bevorzugt von 2,5-35 kN angewandt. Als Beispiel für geeignete Tablettenpressen können die Presse Fette^{®} 102i (Fette GmbH, Deutschland) genannte werden. Im Falle von Exzenterpressen wird üblicherweise eine Presskraft von 1-20 kN, bevorzugt von 2,5-10 kN angewandt. Als Beispiel für eine geeignete Exzenterpresse kann das Handelsprodukt Korsch^{®} EKO genannt werden.

Das Verpressen zu Tabletten erfolgt erfindungsgemäß bevorzugt in Abwesenheit von Lösemitteln, insbesondere von organischen Lösemitteln, das heißt als Trockenkompression.

Sollen die erfindungsgemäßen Arzneimittel in Form von Tabletten vorliegen, die unzerkaut geschluckt werden, ist es bevorzugt, diese Tabletten mit einer Filmschicht zu überziehen. Die vorstehend genannten Anteile an Wirkstoff und Hilfsstoffen beziehen sich auf die unbeschichtete Tablette. Für die Befilmung werden bevorzugt makromolekulare Stoffe verwendet, beispielsweise modifizierte Cellulose, Polymethacrylate, Polyvinylpyrrolidon, Polyvinylacetatphthalat, Zein, Eudragit und/oder Schellack. Es ist erfindungsgemäß ebenfalls möglich, die Tabletten mit einer enterischen Beschichtung zu versehen. Enterische Beschichtungsmaterialien sind dem Fachmann bekannt.

Die Schichtdicke des Tablettenüberzugs beträgt bevorzugt 2-100 µm.

Die erfindungsgemäßen Tabletten weisen bevorzugt ein Verhältnis von Tablettenhöhe zu Gewicht im Bereich von 0,005-0,3 mm/mg, besonders bevorzugt von 0,05-0,2 mm/mg auf.

Das erfindungsgemäße Verfahren wird bevorzugt so ausgeführt, dass das erfindungsgemäße Arzneimittel Silodosin in einer Menge von 0.5 mg bis 15 mg, mehr bevorzugt von 2 mg bis 11 mg , insbesondere von 4 mg bis 8 mg enthält. Gegenstand der Erfindung sind somit z.B. Arzneimittel, insbesondere Tabletten, enthaltend 4 mg oder 8 mg Silodosin.

Erfindungsgemäß besonders vorteilhaft ist auch, dass die mit dem erfindungsgemäßen Verfahren hergestellten Arzneimittel und insbesondere die erfindungsgemäßen Tabletten eine hervorragende content uniformity aufweisen, die im Bereich von 95-105% des durchschnittlichen Gehaltes liegt, bevorzugt im Bereich von 98-102% des durchschnittlichen Gehalts, besonders bevorzugt im Bereich von 99-101% vom durchschnittlichen Gehalt. Die content uniformity wird gemäß PH Eur. 6.0 Abschnitt 2.9.6 bestimmt.

Die erfindungsgemäßen Tabletten weisen ein besonders vorteilhaftes schnelles Freisetzungsprofil auf. Besonders bevorzugt ist das Freisetzungsprofil der erfindungsgemäßen Tabletten gemäß der USP-Methode (Paddle, 900 ml, 0,1 normale HCl, pH 1,2, 37°C, 75 rpm) so, dass nach 10 Minuten üblicherweise mindestens 40%, bevorzugt mindestens 60%, insbesondere mindestens 85% des Wirkstoffs freigesetzt sind. Das Freisetzungsprofil der erfindungsgemäßen Tabletten ändert sich während der Lagerung nicht wesentlich, was ebenfalls ein besonderer Vorteil der erfindungsgemäßen Tabletten darstellt.

Erfindungsgemäß besonders bevorzugt ist ein Verfahren zur Herstellung eines oralen Arzneimittels umfassend die Schritte:
a) Einbringen von Silodosin oder einem pharmazeutisch verträglichen Salz davon und Cyclodextrin in Ethanol oder Ethanol/Wasser, gegebenenfalls Zufügen eines Tensids,
b) Homogenisieren des Gemisches unter Bildung einer Einschlussverbindung mit dem Cyclodextrin wobei eine Lösung oder homogene Suspension entsteht,
c) Die Lösung bzw. Suspension wird auf ein Füllmittel gegeben, das anschließend granuliert wird,
d) Abziehen des Lösemittels bis auf einen Bereich von bevorzugt 0,5%, stärker bevorzugt 0,3% Restfeuchtigkeit, z.B. im Trockenschrank oder durch Sprühtrocknung,
e) Sieben des getrockneten Granulats,
f) Zusatz weiterer handelsüblicher pharmazeutischer Hilfsstoffe zur Herstellung eines fließfähigen Granulats,
g) gegebenenfalls Zusatz weiterer pharmazeutischer Hilfsstoffe und Abfüllen in eine Hartgelatinekapsel oder Verpressen des Granulats und der gegebenenfalls vorhandenen Hilfsstoff zu einer Tablette und
h) gegebenenfalls Befilmen der Tablette.

Erfindungsgemäß ebenfalls bevorzugt ist ein Verfahren zur Herstellung eines oralen Arzneimittels umfassend die Schritte:
a) Mischen von Cyclodextrin mit einem Lösemittel, insbesondere Wasser bei einer erhöhten Temperatur von 30 bis 70°C, insbesondere 40 bis 60°C zu einer Paste,
b) Zugabe von Silodosin zu der Paste und Homogenisieren der Paste in dem Knetmischer für einen Zeitraum von mindestens zwei Stunden, bevorzugt mindestens drei Stunden, z.B. etwa vier Stunden,
c) Trocknen der homogenisierten Paste auf den gewünschten Restlösemittelgehalt,
d) Sieben der getrockneten Paste auf eine gewünschte Teilchengröße,
e) Zugabe der getrockneten und gesiebten Paste zu einem Füllmittel, insbesondere mikrokristalline Cellulose,
f) Zugabe weiterer üblicher pharmazeutisch verträglicher Zusatzmittel,
g) Mischen der getrockneten gesiebten Paste mit den Zusatzstoffen, bis man eine einheitliche Mischung erhält,
h) Abfüllen des Gemisches in eine Hartgelatinekapsel oder Verpressen des Gemisches zu einer Tablette.

Erfindungsgemäß erfolgt das Homogenisieren des Cyclodextrins und des Silodosins in dem Gemisch bzw. der Paste zur Bildung der Silodosin-Cyclodextrin Einschlussverbindung, bis der gewünschte Anteil an Silodosin-Cyclodextrin Einschlussverbindung erhalten wurde. Bevorzugt erfolgt das Homogenisieren über einen Zeitraum von mindestens einer Stunde, stärker bevorzugt mindestens zwei Stunden, noch stärker bevorzugt mindestens drei Stunden, am stärksten bevorzugt vier Stunden oder mehr. Wird die Silodosin Einschlussverbindung in einer Paste gebildet, erfolgt das Homogenisieren bevorzugt mindestens über einen Zeitraum von zwei Stunden, stärker bevorzugt mindestens über einen Zeitraum von drei Stunden, am stärksten bevorzugt über einen Zeitraum von vier Stunden oder mehr.

Die folgenden Beispiele erläutern die Erfindung.

### Beispiel 1

4 g Silodosin (freie Base), 8 g γ-Cyclodextrin und 0,15 g Natriumdodecylsulfat wurden in 30 ml Ethanol eingebracht. Es wurde 240 Minuten mit einem Magnetrührer bei einer Rührgeschwindigkeit von 400 rpm homogenisiert, wodurch sich der Cyclodextrin-Silodosin-Komplex bildete, der in dem Lösemittel gelöst vorliegt.

Das Ethanol mit dem gelösten Komplex wurde auf 150 g Calciumhydrogenphosphat gegeben und es wurde einem Diosna P1/6 Granulator granuliert. Das Granulat wurde 5 Stunden in einem Hordentrockner bei 25°C getrocknet und anschließend auf eine Teilchengröße von 750 µm gesiebt.

Zu dem Gemisch wurden 10 g quervernetztes PVP der Marke Kollidon CL, BASF und 0,1 g Magnesiumstearat gegeben und es wurde 5 Minuten in einem Turbula W10B Mischer gemischt.

Das Gemisch wurde getrennt, eine Hälfte des Gemisches wurde in Kapseln der Größe 3 mit einer 4 mg Dosierung abgefüllt, die zweite Hälfte des Gemischs wurde trocken zu

Tabletten mit einer Dosierung von 4 mg verpresst.

### Beispiel 2

8 g Silodosin und 8 g γ-Cyclodextrin wurden in 80 ml eines Gemisches aus 70% Ethanol und 30% Wasser gegeben. Das Gemisch wurde durch Rühren mit einem Magnetrührer für 300 Minuten bei einer Rührgeschwindigkeit von 400 rpm homogenisiert, wodurch sich der Cyclodextrinkomplex bildete. Das Gemisch wurde auf 250 g Sojapolysaccharid der Marke Emcosoy^{®} gegeben in einem Diosna P1/6 Granulator granuliert. Das Granulat wurde 5 Stunden im Hordentrockner bei 25°C getrocknet und anschließend auf eine Teilchengröße von 500 µm gesiebt.

Zu dem Gemisch wurden 10 g quervernetztes PVP der Marke Kollidon CL, BASF 0,15 g Magnesiumstearat und 0,15 Natriumdodecylsulfat zugegeben. Das Gemisch wurde 5 Minuten in einem Turbula W10B Mischer gemischt und in zwei Teile geteilt. Die eine Hälfte wurde in Kapseln der Größe 1 mit 8 mg Dosierung abgefüllt, die andere Hälfte wurde trocken zu Tabletten mit einer Dosierung von 8 mg verpresst.

### Beispiel 3

8 g Cyclodextrin wurde mit 6 g Wasser in einem Knetmischer, Diosna P1/6 bei einer Umdrehungszahl von 500 rpm zu einer Paste verarbeitet. Es wurde 3,0 g Silodosin zu Paste zugegeben und für weitere 4 Stunden bei einer Umdrehungszahl von 500 rpm durchgeknetet. Die Trocknung der Knetmasse erfolgte unter Vakuum bei einer Temperatur von 30°C über 2 Stunden. Nach der Trocknung wurde die Mischung über ein Sieb der Maschenweite 500 µg gegeben und zu einer Mischung aus 110 g silifizierte mikrokristalline Cellulose (Prosolv) mit 0,1 g Natriumdodecylsulfat und 5 g Talg gegeben. Die Mischung wurde anschließend für 10 Minuten in einem Freifallmischer (Turbula W10B) gemischt. Die eine Hälfte wurde in Hartgelatinekapseln der Größe 1 mit 8 mg Dosierung abgefüllt, die andere Hälfte wurde trocken zu Tabletten mit einer Dosierung von 8 mg verpresst.

## Patentansprüche

1. Verfahren zur Herstellung eines Arzneimittels mit einer Einschlussverbindung aus dem Wirkstoff Silodosin oder einem pharmazeutisch verträglichen Salz davon und einem Cyclodextrin, umfassend die Verfahrensschritte
a) Silodosin oder ein pharmazeutisch verträgliches Salz davon wird mit einem Cyclodextrin in Gegenwart eines Lösemittels in Kontakt gebracht, wodurch eine Silodosin-Cyclodextrin Einschlussverbindung entsteht,
b) die Silodosin-Cyclodextrin Einschlussverbindung aus Schritt a) wird mit einem oder mehreren pharmazeutisch verträglichen Zusatzstoffen gemischt,
c) Trocknen des Produktes aus Schritt a) und/oder aus Schritt b) und
d) gegebenenfalls wird das getrocknete Gemisch gegebenenfalls mit weiteren pharmazeutisch verträglichen Zusatzstoffen zu einer Einheitsdosisform weiterverarbeitet.

2. Verfahren nach Anspruch 1, bei dem
a) Silodosin oder ein pharmazeutisch verträgliches Salz davon, Cyclodextrin und gegebenenfalls ein Tensid in ein Lösemittel eingebracht werden, wodurch ein Gemisch entsteht,
b) das Gemisch aus Schritt a) homogenisiert wird, wodurch eine Silodosin-Cyclodextrin Einschlussverbindung entsteht,
c) das homogenisierte Gemisch auf ein Füllmittel aufgebracht wird,
d) das Füllmittel mit dem homogenisierten Gemisch und gegebenenfalls weiteren pharmazeutisch verträglichen Zusatzstoffen granuliert wird,
e) das erhaltene Granulat getrocknet wird,
f) das getrocknete Granulat gegebenenfalls zerkleinert und/oder gesiebt wird und
g) gegebenenfalls zu einer Einheitsdosisform weiterverarbeitet.

3. Verfahren nach Anspruch 1, bei dem
a) Cyclodextrin mit einem Lösemittel zu einer Paste verarbeitet wird,
b) Silodosin oder ein pharmazeutisch verträgliches Salz davon in diese Paste eingebracht wird,
c) die Paste homogenisiert wird, wodurch die Silodosin-Cyclodextrin Einschlussverbindung entsteht,
d) die homogenisierte Paste getrocknet und gesiebt wird,
e) mit mindestens einem pharmazeutisch verträglichen Zusatzstoff gemischt wird und
f) gegebenenfalls das Gemisch zu einer Einheitsdosisform weiterverarbeitet wird.

4. Verfahren nach einem der Ansprüche 1 bis 3, **dadurch gekennzeichnet, dass** es sich bei dem Cyclodextrin um γ-Cyclodextrin handelt.

5. Verfahren nach einem der Ansprüche 1 bis 4, **dadurch gekennzeichnet, dass** das Mol-Verhältnis von Cyclodextrin zu Silodosin bzw. zu dem pharmazeutisch verträglichen Salz des Silodosins im Bereich von 1:1 1 bis 10:1 liegt.

6. Verfahren nach einem der Ansprüche 1 bis 5, **dadurch gekennzeichnet, dass** es sich bei dem Lösemittel in Schritt a) um einen Alkohol mit 1 bis 4 Kohlenstoffatomen, um Wasser oder um ein Gemisch aus Wasser mit einem Alkohol mit 1 bis 4 Kohlenstoffatomen handelt.

7. Verfahren nach Anspruch 2, **dadurch gekennzeichnet, dass** es sich bei dem Füllmittel um eine Verbindung ausgewählt aus Calciumcarbonat, Magnesiumcarbonat, Magnesiumoxid, Calciumsulfat, Stärke, Stärkederivaten, modifizierter Stärke, Polysacchariden, Chitin, Cellulose, Cellulosederivaten, Calciumphosphat, Calciumhydrogenphosphat, Saccharose, Maltodextrin, Dextrat, Dextrin, Dextrose, Laktose, Laktosederivaten und hydrogeniertem Pflanzenöl handelt.

8. Verfahren nach einem der Ansprüche 1 bis 7, **dadurch gekennzeichnet, dass** das Trocknen auf eine Restfeuchte von 0,5% oder weniger erfolgt.

9. Arzneimittel mit einer Einschlussverbindung aus dem Wirkstoff Silodosin mit einem Cyclodextrin, erhältlich nach einem Verfahren nach einem der Ansprüche 1 bis 8.

10. Arzneimittel nach Anspruch 9, bei dem mindestens 85% des vorhandenen Silodosins als Cyclodextrineinschlussverbindung vorliegt.

11. Arzneimittel nach Anspruch 9 oder 10, bei dem es sich um eine Tablette oder um eine Hartgelatinekapsel handelt.

12. Arzneimittel nach einem der Ansprüche 9 bis 11 mit folgenden Bestandteilen:
1-5 Gew,-% Silodosin oder ein pharmazeutisch verträgliches Salz davon
1-50 Gew.-% Cyclodextrin
40-94,5 Gew.-% Füllmittel
3-6 Gew.-% Sprengmittel
0,5-4 Gew.-% Schmiermittel.

## Claims

1. A process for the preparation of a medicinal drug with an inclusion compound of the active substance silodosin or a pharmaceutically acceptable salt thereof and a cyclodextrin, which comprises the process steps of
a) contacting silodosin or a pharmaceutically acceptable salt thereof with a cyclodextrin in the presence of a solvent to form a silodosin-cyclodextrin inclusion compound,
b) mixing the silodosin-cyclodextrin inclusion compound of step a) with one or more pharmaceutically acceptable additives,
c) drying the product of step a) and/or of step b); and
d) optionally further processing the dried mixture with optional additional pharmaceutically acceptable additives into a unit dosage form.

2. The process according to claim 1, wherein
a) silodosin or a pharmaceutically acceptable salt thereof, cyclodextrin, and optionally a surfactant are introduced into a solvent to form a mixture,
b) the mixture of step a) is homogenized to form a silodosin-cyclodextrin inclusion compound,
c) the homogenized mixture is applied to a filler,
d) the filler is granulated with the homogenized mixture and optionally additional pharmaceutically acceptable additives,
e) the obtained granulate is dried,
f) the dried granulate is optionally comminuted and/or sieved; and
g) is optionally further processed into a unit dosage form.

3. The process according to claim 1, wherein
a) cyclodextrin is processed with a solvent into a paste,
b) silodosin or a pharmaceutically acceptable salt thereof is introduced into said paste,
c) the paste is homogenized to form the silodosin-cyclodextrin inclusion compound,
d) the homogenized paste is dried and sieved,
e) is mixed with at least one pharmaceutically acceptable additive; and
f) optionally the mixture is further processed into a unit dosage form.

4. The process according to anyone of claims 1 to 3, **characterized in that** the cyclodextrin is γ-cyclodextrin.

5. The process according to anyone of claims 1 to 4, **characterized in that** the molar ratio of cyclodextrin to silodosin or to the pharmaceutically acceptable salt of the silodosin, respectively, is in the range of from 1:1 to 10:1.

6. The process according to anyone of claims 1 to 5, **characterized in that** the solvent in step a) is an alcohol with 1 to 4 carbon atoms, water or a mixture of water with an alcohol with 1 to 4 carbon atoms.

7. The process according to claim 2, **characterized in that** the filler is a compound selected from calcium carbonate, magnesium carbonate, magnesium oxide, calcium sulfate, starch, starch derivatives, modified starch, polysaccharides, chitin, cellulose, cellulose derivatives, calcium phosphate, calcium hydrogen phosphate, sucrose, maltodextrin, dextrat, dextrin, dextrose, lactose, lactose derivatives, and hydrogenated vegetable oil.

8. The process according to anyone of claims 1 to 7, **characterized in that** drying is carried out to a residual moisture of 0.5% or less.

9. A medicinal drug with an inclusion compound of the active substance silodosin with a cyclodextrin that is obtained according to a process according to anyone of claims 1 to 8.

10. The medicinal drug according to claim 9 wherein at least 85% of the present silodosin are present as a cyclodextrin inclusion compound.

11. The medicinal drug according to claim 9 or 10 that is a tablet or a hard gelatin capsule.

12. The medicinal drug according to anyone of claims 9 to 11 having the following ingredients:
1-5% by weight of silodosin or a pharmaceutically acceptable salt thereof
1-50% by weight of cyclodextrin
40-94.5% by weight of a filler
3-6% by weight of disintegrant
0.5-4% by weight of a lubricant.

## Revendications

1. Procédé de production d'un médicament avec un composé d'inclusion du principe actif silodosine ou d'un de ses sels pharmaceutiquement acceptables et d'une cyclodextrine, comprenant les étapes
a) la silodosine ou un de ses sels pharmaceutiquement acceptables est mis en contact avec une cyclodextrine en présence d'un solvant, ce qui donne un composé d'inclusion silodosine-cyclodextrine,
b) le composé d'inclusion silodosine-cyclodextrine de l'étape a) est mélangé avec un ou plusieurs additifs pharmaceutiquement acceptables,
c) le produit de l'étape a) et/ou de l'étape b) est séché, et
d) le mélange séché est éventuellement traité ultérieurement avec d'autres additifs pharmaceutiquement acceptables pour donner une forme galénique unitaire.

2. Procédé selon la revendication 1, selon lequel
a) la silodosine ou un de ses sels pharmaceutiquement acceptables, la cyclodextrine et éventuellement un tensioactif sont introduits dans un solvant, ce qui donne un mélange,
b) le mélange de l'étape a) est homogénéisé, ce qui donne un composé d'inclusion silodosine-cyclodextrine,
c) le mélange homogénéisé est appliqué sur une matière de charge,
d) la matière de charge avec le mélange homogénéisé et éventuellement d'autres additifs pharmaceutiquement acceptables est granulée,
e) les granulés sont séchés,
f) les granulés séchés sont éventuellement broyés et/ou criblés, et
g) éventuellement transformés en une forme galénique unitaire.

3. Procédé selon la revendication 1, selon lequel
a) la cyclodextrine est transformée avec un solvant en une pâte,
b) la silodosine ou un de ses sels pharmaceutiquement acceptables est introduit dans cette pâte,
c) la pâte est homogénéisée, ce qui donné le composé d'inclusion silodosine-cyclodextrine,
d) la pâte homogénéisée est séchée et criblée,
e) est mélangée avec un moins un additif pharmaceutiquement acceptable, et
f) le mélange est éventuellement transformé en une forme galénique unitaire.

4. Procédé selon l'une quelconque des revendications 1 à 3, **caractérisé en ce que** la cyclodextrine est de la γ-cyclodextrine.

5. Procédé selon l'une quelconque des revendications 1 à 4, **caractérisé en ce que** le rapport molaire de la cyclodextrine à la silodosine ou encore au sel pharmaceutiquement acceptable de la silodosine est de l'ordre de 1 :1 à 10 :1.

6. Procédé selon l'une quelconque des revendications 1 à 5, **caractérisé en ce que** le solvant à l'étape a) est un alcool ayant de 1 à 4 atomes de carbone, d'eau ou d'un mélange d'eau avec un alcool ayant de 1 à 4 atomes de carbone.

7. Procédé selon la revendication 2, **caractérisé en ce que** la matière de charge est un composé sélectionné dans le groupe consistant en carbonate de calcium, carbonate de magnésium, oxyde de magnésium, sulfate de calcium, amidon, dérivés d'amidon, amidon modifié, polysaccharides, chitine, cellulose, dérivés de cellulose, phosphate de calcium, hydrogénophosphate de calcium, saccharose, maltodextrine, dextrate, dextrine, dextrose, lactose, dérivés de lactose et huile végétale hydrogénée.

8. Procédé selon l'une quelconque des revendications 1 à 7, **caractérisé en ce que** le séchage est effectué jusqu'à obtenir une humidité résiduelle de 0,5 % ou moins.

9. Médicament avec un composé d'inclusion du principe actif silodosine avec une cyclodextrine, obtensible selon le procédé selon l'une quelconque des revendications 1 à 8.

10. Médicament selon la revendication 9, dans lequel au moins 85 % de la silodosine présente est présente comme composé d'inclusion de cyclodextrine.

11. Médicament selon la revendication 9 ou 10, le médicament étant un comprimé ou une capsule de gélatine dure.

12. Médicament selon l'une quelconque des revendications 9 à 11, comprenant les éléments constitutifs suivants :
1-5 % en poids de silodosine ou d'un de ses sels pharmaceutiquement acceptables
1-50 % en poids de cyclodextrine,
40-94,5 % en poids de matière de charge
3-6 % en poids de délitant
0,5-4 % en poids de lubrifiant.
